(19) Europäisches Patentamt
European Patent Office
Office européen des brevets

(11) **EP 2 912 001 B1**

(12) **EUROPÄISCHE PATENTSCHRIFT**

(45) Veröffentlichungstag und Bekanntmachung des Hinweises auf die Patenterteilung:
**14.11.2018 Patentblatt 2018/46**

(21) Anmeldenummer: **13773606.2**

(22) Anmeldetag: **30.09.2013**

(51) Int Cl.:
*C07C 29/141* (2006.01)      *C07C 31/20* (2006.01)
*C07C 45/75* (2006.01)       *C07C 47/19* (2006.01)
*B01J 23/889* (2006.01)      *B01J 35/02* (2006.01)
*B01J 37/03* (2006.01)       *B01J 37/04* (2006.01)
*B01J 37/08* (2006.01)

(86) Internationale Anmeldenummer:
**PCT/EP2013/002930**

(87) Internationale Veröffentlichungsnummer:
**WO 2014/067602 (08.05.2014 Gazette 2014/19)**

(54) **KONTINUIERLICHES VERFAHREN ZUR HERSTELLUNG VON NEOPENTYLGLYKOL**

CONTINUOUS METHOD FOR THE PRODUCTION OF NEOPENTYL GLYCOL

PROCÉDÉ DE PRÉPARATION EN CONTINU DE NÉOPENTYLGLYCOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.10.2012 DE 102012021276**

(43) Veröffentlichungstag der Anmeldung:
**02.09.2015 Patentblatt 2015/36**

(73) Patentinhaber: **OXEA GmbH**
**46147 Oberhausen (DE)**

(72) Erfinder:
• **EISENACHER, Matthias**
  **46485 Wesel (DE)**
• **SCHALAPSKI, Kurt**
  **46147 Oberhausen (DE)**
• **HEYMANNS, Peter**
  **45147 Essen (DE)**
• **LUKAS, Rainer**
  **45133 Essen (DE)**
• **STRUTZ, Heinz**
  **47445 Moers (DE)**

(56) Entgegenhaltungen:
EP-A1- 0 006 460      GB-A- 2 482 887
US-A- 4 855 515

EP 2 912 001 B1

**Beschreibung**

Die vorliegende Erfindung betrifft ein kontinuierliches Verfahren zur Herstellung von Neopentylglykol durch Hydrierung von Hydroxypivalinaldehyd in der Gasphase an barium- und mangandotierten Kupferchromit-Katalysatoren.

Mehrwertige Alkohole oder Polyole besitzen als Kondensationskomponente zum Aufbau von Polyestern oder Polyurethanen, Kunstharzlacken, Schmiermitteln und Weichmachern eine erhebliche wirtschaftliche Bedeutung. Hierbei sind besonders solche mehrwertigen Alkohole von Interesse, die durch eine gemischte Aldoladdition von Formaldehyd mit iso- oder n-Butyraldehyd erhalten werden. Bei der Aldoladdition zwischen Formaldeyd und dem entsprechenden Butyraldehyd bildet sich zunächst eine aldehydische Zwischenstufe, die anschließend zum mehrwertigen Alkohol reduziert werden muss. Ein technisch bedeutender mehrwertiger Alkohol, der nach diesem Verfahren zugänglich ist, ist Neopentylglykol [NPG, 2,2-Dimethylpropandiol-(1,3)] aus der Mischaldolisierung von Formaldehyd und iso-Butyraldehyd.

Die Aldoladdition wird in Gegenwart basischer Katalysatoren beispielsweise Alkalihydroxiden oder aliphatischen Aminen durchgeführt und ergibt zunächst das isolierbare Zwischenprodukt Hydroxypivalinaldehyd (HPA). Dieses Zwischenprodukt kann anschließend mit überschüssigem Formaldehyd entsprechend der Cannizzaro-Reaktion in Neopentylglykol unter Bildung eines Äquivalents eines Formiatsalzes überführt werden. Bei dieser Ausgestaltung des Reduktionsschrittes fällt daher das Formiatsalz als Koppelprodukt an und die Wirtschaftlichkeit dieser Verfahrensvariante hängt auch von den Vermarktungschancen des Formiatsalzes ab.

Technisch umgesetzt wird aber auch die katalytische Hydrierung von Hydroxypivalinaldehyd in der Gas- und Flüssigphase am Metallkontakt. Bei der Gasphasenvariante wird Hydroxypivalinaldehyd in einem der Hydrierstufe vorgeschalteten Verdampfer zunächst von Hochsiedern befreit. Die nachfolgende Hydrierung erfolgt vorzugsweise in Gegenwart von Raney-NickelKatalysatoren oder Trägerkatalysatoren auf Basis von Nickel, die zusätzlich noch weitere aktive Metalle wie Kupfer oder Chrom und darüber hinaus noch Aktivatoren enthalten können. Auf die Gasphasenvariante wird beispielsweise in EP 0 278 106 A1; US 4,094,014; Ullmann's Encyclopedia of Industrial Chemistry, VCH Verlagsgesellschaft, 5th Ed., 1985, Vol. A1, p. 308; Chemiker-Zeitung, 100. Jahrgang (1976), Nr. 12, Seiten 504-514 eingegangen.

Die Hydrierung in der Flüssigphase wird vielfach beschrieben, beispielsweise in der EP 0 484 800 A2 unter Verwendung von Katalysatoren auf Basis von Kupfer, Zink und Zirkonium. Häufig erfolgt die Flüssigphasenhydrierung von Hydroxypivalinaldehyd in Gegenwart von Kupferchromit-Katalysatoren. Kupferchromit-Katalysatoren enthalten als Aktivatoren häufig noch weitere Metalle, beispielsweise Barium, Cadmium, Magnesium, Mangan und/oder ein seltenes Erdmetall. Gemäß US 4,855,515 zeichnen sich besonders mangandotierte Kupferchromit-Katalysatoren bei der Hydrierung des Aldolisierungsproduktes aus der Umsetzung von Formaldehyd mit iso-Butyraldehyd aus. WO98/29374 A1 offenbart die Verwendung eines bariumdotierten Kupferchromit-Katalysators für die Hydrierung von Hydroxypivalinaldehyd in einer methanolischen Lösung.

Nach der Lehre von DE 1 518 784 A1 wird ein Gemisch aus Hydroxypivalinaldehyd und überschüssigem iso-Butyraldehyd zu Neopentylglykol und iso-Butanol in Gegenwart eines Kupferchromit-Katalysators hydriert, der mit Barium dotiert ist. Auch das zweistufige Hochdruckhydrierverfahren von rohem Hydroxypivalinaldehyd nach EP 0 006 460 A1, bei dem mit ansteigenden Hydriertemperaturen gearbeitet wird, verwendet einen mit Barium aktivierten Kupferchromit-Katalysator.

GB 2 482 887 A offenbart einen Kupferchromit-Katalysator zur Hydrierung von Aldehyden zu Alkoholen, der zusätzlich mit Mangan und Barium dotiert ist.

Aus EP 0 522 368 A1 ist bekannt, die Hydrierung von Hydroxypivalinaldehyd in einer Lösung vorzunehmen, die mindestens 20 Gew.-% eines niederen Alkohols beispielsweise Methanol oder n-Butanol, bezogen auf die Mischung aus Alkohol und Reaktionsprodukt, sowie Wasser in einer Menge von nicht mehr als 40 Gew.-%, bezogen auf die Gesamtmenge aus Wasser, Alkohol und Reaktionsprodukt, enthält. Als Hydrierkatalysator wird ein Kupferchromit-Katalysator empfohlen.

Neopentylglykol besitzt als technisch hergestelltes Produkt eine große wirtschaftliche Bedeutung und es besteht daher ein Bedarf, die bekannten Verfahren zur Neopentylglykolherstellung stets zu verbessern, sei es durch eine verbesserte Produktausbeute, durch eine bessere Anlagenausnutzung oder durch einen geringeren Energieeinsatz.

Es wurde überraschenderweise gefunden, dass Neopentylglykol durch Hydrierung von Hydoxypivalinaldehyd in hoher Selektivität und Ausbeute hergestellt werden kann, wenn die Hydrierung kontinuierlich in der Gasphase in Gegenwart eines Kupferchromit-Katalysators erfolgt, der sowohl mit Mangan als auch mit Barium dotiert ist.

Die vorliegende Erfindung betrifft daher ein kontinuierliches Verfahren zur Herstellung von Neopentylglykol durch Addition von iso-Butyraldehyd und Formaldehyd in Gegenwart eines tertiären Alkylamins als Katalysator zum Hydroxypivalinaldehyd mit nachfolgender Hydrierung in der Gasphase bei einer Temperatur von 125 bis 180°C, dadurch gekennzeichnet, dass die Hydrierung in Gegenwart eines Kupferchromit-Katalysators enthaltend die Aktivatoren Barium und Mangan und bei einem Überdruck von 30 bis 120 kPa erfolgt.

Überraschenderweise wurde gefunden, dass bei Verwendung von Kupferchromit-Katalysatoren, die Barium und Mangan als Aktivatoren enthalten, und beim Einstellen einer Hydriertemperatur von 125 bis 180°C, vorzugsweise von 140 bis 180°C die selektive Hydrierung von Hydroxypivalinaldehyd zu Neopentylglykol gelingt.

Bei zu niedrigen Hydriertemperaturen erfolgt die Hydrierung von Hydroxypivalinaldehyd nur unvollständig. Bei zu hohen Hydriertemperaturen tritt ebenfalls verstärkt eine Zersetzung des als Aldolisierungskatalysator eingesetzten tertiären Alkylamins ein, die zu schwer abtrennbaren Folgeprodukten führt und daher unerwünscht ist.

Es ist weiterhin sehr überraschend, dass die Hydrierung bei einem äußerst geringen Überdruck von nur 30 bis 120 kPa mit hoher Ausbeute zum Neopentylglykol gelingt. Das erfindungsgemäße Verfahren erfordert daher nur einen sehr geringen Aufwand an Kompressorleistung und ist daher sehr energiesparend.

Die Aldoladdition von iso-Butyraldehyd und einer wässrigen Formaldehydlösung erfolgt in Gegenwart von tertiären Alkylaminen als Aldoladditionskatalysator, die gleiche oder unterschiedliche Alkylgruppen enthalten können und somit symmetrisch oder unsymmetrisch aufgebaut sein können, sowie in Gegenwart von tertiären Alkylaminen mit mehreren Trialkylaminfunktionen. Beispielsweise wird in Gegenwart von Trimethyl-, Triethyl-, Tri-n-propyl-, Triiso-propyl-, Methyl-diethyl-, Methyl-diisopropylamin, Tri-n-butylamin, Dimethyl-tert-butylamin oder N,N'-Tetramethylethylendiamin gearbeitet. Als besonders geeignete Katalysatoren haben sich Trimethylamin, Triethylamin, Tri-n-propylamin und Tri-n-butylamin bewährt.

Die Aldehyde können im molaren Verhältnis umgesetzt werden, jedoch ist es auch möglich, einen der beiden Reaktionspartner im Überschuss anzuwenden. Man setzt Formaldehyd als wässrige Lösung ein, der Formaldehydgehalt beträgt üblicherweise von 20 bis 50 Gew.-%. Es hat sich herausgestellt, dass der in dem erfindungsgemäßen Verfahren verwendete dotierte Kupferchromit-Katalysator eine überraschend hohe Resistenz gegenüber Formaldehyd besitzt. Daher können in der Aldoladditionsstufe Molverhältnisse von Formaldehyd zu iso-Butyraldehyd von 1:1 bis zu Gunsten von Formaldehyd, im Allgemeinen bis zu 1,2:1, vorzugsweise bis 1,1:1 eingestellt werden. Durch die Verringerung des iso-Butyraldehydeinsatzes wird die iso-Butanolbildung in der Hydrierstufe zurückgedrängt und die Neopentylglykolausbeute, bezogen auf iso-Butyraldehydeinsatz, erhöht.

Die Reaktion zwischen iso-Butyraldehyd und Formaldehyd erfolgt bei Temperaturen zwischen 20 und 100°C, zweckmäßig arbeitet man bei 80 bis 95°C. Im Allgemeinen wird die Reaktion bei Normaldruck durchgeführt, jedoch kann man auch erhöhten Druck anwenden. Das als Aldoladditionskatalysator verwendete tertiäre Alkylamin ist in dem Reaktionsgemisch in einer Menge von 1 bis 20, vorzugsweise 2 bis 12 Mol.-%, bezogen auf iso-Butyraldehyd, enthalten.

Neben dem Wasser aus der wässrigen Formaldehydlösung und geringen Anteilen an Methanol, das ebenfalls in der wässrigen Formaldehydlösung enthalten ist, wird gegebenenfalls der Reaktionsmischung noch iso-Butanol als Verdünnungsmittel zugesetzt. Der iso-Butanolzusatz ist nicht zwingend erforderlich, falls jedoch iso-Butanol zugesetzt wird, liegt sein Gehalt in der Reaktionsmischung im Bereich von 15 bis 30 Gew.-%, vorzugsweise 15 bis 25 Gew.-%, bezogen auf den organischen Anteil im gesamten Reaktionsgemisch. Weitere Lösungs- und Verdünnungsmittel sind nicht erforderlich. Die praktische Durchführung der Additionsreaktion erfolgt in einem Rührkessel, in einer Rührkesselkaskade oder in einem Reaktionsrohr, das zur besseren Durchmischung der Reaktanten mit Füllkörpern oder anderen Einbauten beschickt sein kann. Die Umsetzung verläuft exotherm, sie kann durch Erhitzen beschleunigt werden.

Das nach der Aldoladdition anfallende Rohgemisch wird, gegebenenfalls nach destillativer oder extraktiver Abtrennung von flüchtigen Bestandteilen wie Wasser, Methanol, Isobutanol und Restmengen an Formaldehyd, Isobutyraldehyd und des Aldolisierungskatalysators oder aber ohne vorherige Auftrennung auf eine Verdampfereinrichtung gegeben, die im Allgemeinen bei Temperaturen von 140 bis 190°C unter Normaldruck betrieben wird. Hochsieder fallen im Verdampfer als Rückstand an und werden aus dem Verfahren ausgeschleust.

Diese Hochsieder können thermisch verwertet werden oder beispielsweise gemäß der Arbeitsweise nach DE 10 2008 033 163 A1 separat hydrierend gespalten werden. Bei den Hochsiedern handelt es sich um sauerstoffhaltige Verbindungen wie Ester oder cyclische Acetale, in denen Äquivalente des Neopentylglykols chemisch gebunden sind. In den Hochsiedern ist der Anteil an Mono- und Di-iso-Buttersäureestern des Neopentylglykols sowie an dem aus Hydroxypivalinaldehyd nach der Tischtschenko-Reaktion gebildeten Disproportionierungsprodukt Neopentylglykolmonohydroxypivalinsäureester besonders hoch.

Der flüchtige Verdampferabzug enthält im Wesentlichen Hydroxypivalinaldehyd und, je nach optionaler Aufarbeitung des rohen Aldoladditionsgemisches, Wasser, das Verdünnungsmittel, falls zugesetzt, weitere flüchtige Anteile, wie Methanol aus der Formaldehydstabilisierung. Ebenfalls sind mitgerissene Reste an Hochsiedern enthalten und das als Aldolisierungskatalysator verwendete tertiäre Amin. Wird das rohe Aldoladditionsgemisch ohne optionale Auftrennung auf den Verdampfer gegeben, enthält der Verdampferabzug im Allgemeinen von 15 bis 30 Gew.-%, vorzugsweise 15 bis 25 Gew.-% Isobutanol, bezogen auf den organischen Anteil im Verdampferabzug.

In einer bevorzugten Ausgestaltung der Erfindung wird im Verdampferabzug der Wassergehalt, bezogen auf den gesamten Verdampferabzug, auf einen Wert von 15 bis 25 Gew.-%, vorzugsweise von 18 bis 25 Gew.-%, eingestellt, so dass die nachfolgende Hydrierstufe in Gegenwart von Wasser in der eingestellten Menge durchgeführt wird. Der Rest auf 100 Gew.-% ist der organische Anteil. Durch die bei der bevorzugten Ausgestaltung der Erfindung eingestellte Wassermenge wird die selektive Spaltung der zuvor erwähnten sauerstoffhaltigen Hochsieder zu Neopentylglykol in der nachfolgenden Hydrierstufe verbessert. Auch fördert der Wasseranteil eine vorteilhafte Wärmeverteilung und ein vorteilhaftes Abführen der Reaktionswärme bei dem Hydrierschritt und mindert die Gefahr des Auftretens von lokalen Temperaturspitzen. Bei einer Arbeitsweise, bei der man einen Verdampferabzug mit einem Wassergehalt von weniger als 15 Gew.-% verwendet und bei der damit die Hydrierung in Gegenwart einer geringeren Wassermenge erfolgt, ist der vorteilhafte Effekt auf die Verminderung des Hochsiederanteils weniger deutlich ausgeprägt. In diesem Falle ist es zu empfehlen, gezielt Wasser auf den erforderlichen Gehalt zuzusetzen. Bei zu hohen Wassergehalten wird wertvolles Reaktorvolumen unnötig belegt und nicht ausgenutzt.

Der erhaltene flüchtige Verdampferabzug wird ohne weitere Reinigungs- und Aufarbeitungsschritte nachfolgend hydriert.

Die Hydrierung des rohen Hydroxypivalinaldehyds wird bei einer Temperatur von 125 bis 180°C, vorzugsweise von 140 bis 180°C, in der Gasphase in Gegenwart von barium- und mangandotierten Kupferchromit-Katalysatoren durchgeführt. Der Überdruck beträgt 30 bis 120 kPa, vorzugsweise 60 bis 100 kPa. Besonders bewährt sich eine Reaktionstemperatur von 140 bis 180°C und ein Überdruck von 60 bis 100 kPa. Bei noch niedrigeren Überdrücken wird keine zufriedenstellende Hydrierung des Hydroxypivalinaldehyds mehr beobachtet.

Die Hydrierung von Hydroxypivalinaldehyd erfolgt in Gegenwart von Kupferchromit-Katalysatoren, die als Aktivatoren Barium und Mangan enthalten. Kupferchromit-Katalysatoren lassen sich nach H. Adkin, Org. React. 8, 1954, 1-27 als äquimolare Kombination von Kupferoxid und Kupferchromit beschreiben, obgleich sie nicht unbedingt Kupferchromit enthalten. Die Katalysatoren können entweder ohne Trägerstoffe als sogenannte Vollkatalysatoren oder mit Trägerstoffen beispielsweise mit Kieselguhr, Kieselgel oder Aluminiumoxid als Pulver oder in Form von Tabletten, Sternen, Strängen, Ringen oder anderen Teilchen von verhältnismäßig großer Oberfläche verwendet werden.

Zur Herstellung werden entweder unlösliche Verbindungen von Kupfer, Chrom, Mangan und Barium gemischt, beispielsweise in angeteigter Form und zu geeigneten Formkörpern wie Strängen oder Tabletten geformt. Nach der Formgebung wird getrocknet und bis zu 500°C calciniert, wobei sich der Feststoff verdichtet und die anwesenden Metalle gegebenenfalls in die Oxide überführt werden.

Ebenfalls kann man von zweckmäßigerweise wässrigen Lösungen ausgehen, aus denen man das Gelöste ausfällt. Nach Filtration wird der Feststoff getrocknet und wie bei festen Mischungen bis zu 500°C calciniert. Anschließend kann es empfehlenswert sein, den Feststoff in einer niederen organischen Säure wie Ameisensäure, Essigsäure, Propionsäure oder n-Buttersäure aufzurühren um lösliche Bestandteile abzutrennen, anschließend säurefrei zu waschen, erneut zu trocknen und bis 500°C zu calcinieren.

Nach Zugabe von Hilfsmitteln wie Graphit oder Alkali- oder Erdalkalimetallseifen können anschließend Formkörper wie Tabletten oder Ringe gefertigt werden.

Die barium- und mangandotierten Kupferchromit-Katalysatoren enthalten von 0,5 bis 8 Gew.-%, vorzugsweise von 3 bis 5 Gew.% Mangan und von 0,5 bis 8 Gew.-%, vorzugsweise von 1 bis 4 Gew.-% Barium, jeweils bezogen auf die Summe des Gehalts an Kupfer, Chrom, Barium und Mangan. Besonders bewährt sich ein Bariumgehalt in einem Bereich von 1 bis 4 Gew.-% und ein Mangangehalt in einem Bereich von 3 bis 5 Gew.-%, jeweils bezogen auf die Summe des Gehalts an Kupfer, Chrom, Barium und Mangan. Neben den genannten Aktivatoren können gegebenenfalls noch weitere Aktivatoren wie Cadmium, Magnesium, Strontium und/oder ein seltenes Erdmetall zugegen sein.

Die Hydrierung wird in der Gasphase kontinuierlich durchgeführt, beispielsweise an fest angeordneten Katalysatoren oder in der Wirbelschicht an fluidisierten Katalysatoren.

Bei der kontinuierlichen Fahrweise hat sich eine Katalysatorbelastung V/Vh, ausgedrückt in Durchsatzvolumen pro Katalysatorvolumen und Zeit, von 0,2 bis 2,0 $h^{-1}$, vorzugsweise 0,3 bis 1,0 $h^{-1}$, als zweckmäßig erwiesen.

Eine höhere Belastung des Kupferchromit-Katalysators ist zu vermeiden, weil die Ausgangsverbindung Hydroxypivalinaldehyd dann nicht mehr vollständig hydriert wird und eine verstärkte Nebenproduktbildung beobachtet wird.

Vorzugsweise wird die Hydrierung kontinuierlich in der Gasphase bei geradem Durchgang durchgeführt. Ebenfalls ist eine Kreisgasfahrweise möglich, bei der sich das Verhältnis von Kreisgasstrom zu Frischgasstrom von 40 bis 70 als zweckmäßig erwiesen hat.

Die Hydrierung erfolgt vorzugsweise mit reinem Wasserstoff. Es können jedoch auch Gemische verwendet werden, die freien Wasserstoff und daneben unter den Hydrierbedingungen inerte Bestandteile enthalten.

Die Gewinnung des reinen Neopentylglykols aus dem hydrierten Reaktionsgemisch erfolgt nach konventionellen Destillationsverfahren. Dabei abgetrenntes Verdünnungsmittel kann wieder in die Aldoladditionsstufe zurückgeführt werden.

Nach dem erfindungsgemäßen Hydrierverfahren wird Hydroxypivalinaldehyd bei hohem Umsatz mit hoher Selektivität in Neopentylglykol umgewandelt. Bemerkenswert ist der sehr geringe Überdruck in der Hydrierung.

Auch wird die Spaltung des tertiären Alkylamins in flüchtige, stickstoffhaltige Verbindungen, die zu unerwünschten Verunreinigungen führen und die bei der anschließenden destillativen Aufarbeitung nur schwierig abzutrennen sind und die bei der Weiterverarbeitung von Neopentylglykol stören, unterdrückt.

Im Folgenden wird das erfindungsgemäße Verfahren anhand einiger Beispiele näher erläutert.

Beispiele

**Beispiel 1: Herstellen eines mangan- und bariumdotierten Kupferchromit-Katalysators**

2,8 kg Kupfernitrat-Trihydrat, 400 g Mangannitrat in Form einer 50%igen Lösung in verdünnter Salpetersäure und 150 g Bariumnitrat wurden in 20 Liter Wasser bei 55°C gelöst. Separat wurden 2,6 kg Ammoniumdichromat in 12 Liter Wasser und 4 Liter 25%iger Ammoniaklösung gelöst. Danach wurde die Ammoniumdichromatlösung langsam in die Kupfernitratlösung getropft. Dabei fiel ein rot-brauner Feststoff aus. Zur Vervollständigung der Fällung wurde noch eine Stunde nachgerührt und dabei auf Raumtemperatur abgekühlt. Anschließend wurde der Feststoff abfiltriert und bei 110°C im Trockenschrank getrocknet. Der getrocknete Feststoff wurde bei 350°C vier Stunden kalziniert, die Aufheizrate betrug dabei 2°C/min. Nach dem Kalzinieren und Erkalten des Feststoffs wurde dieser mit 20 Liter 10%iger Essigsäure aufgerührt. Danach wurde der Feststoff mit Wasser säurefrei gewaschen und erneut bei 110°C getrocknet und bei 350°C mit einer Aufheizrate von 2°C/min geglüht. Der erhaltene Feststoff konnte in dieser Form als Katalysator eingesetzt werden. Auf die Metalle bezogen wies der Katalysator folgende Zusammensetzung auf: 47,5% Kupfer, 46,5% Chrom, 4,0% Mangan, 2,0% Barium.

**Beispiel 2: Einsatz des Katalysators aus Beispiel 1 als Festbettkatalysator**

Der Katalysator aus Beispiel 1 wurde mit 3% Graphit vermischt und zu 5x5 mm Tabletten geformt. Der tablettierte Katalysator wurde in einen 2,5 Liter Rohrreaktor gefüllt und anschließend unter folgenden Bedingungen in drei Stufen aktiviert:

| | |
|---|---|
| Aufheizrate: | 20°C/h bis 180 °C |
| Stickstoffzufuhr: | 1000 NL/h |
| Wasserstoffzufuhr: | 20 NL/h |
| Dauer: | 12 Stunden |
| | |
| Stickstoffzufuhr: | 1000 NL/h |
| Wasserstoffzufuhr: | 60 NL/h |
| Dauer: | 6 Stunden |
| | |
| Stickstoffzufuhr: | 1000 NL/h |
| Wasserstoffzufuhr: | 120 NL/h |
| Dauer: | 6 Stunden |

NL = Normliter, 1 Liter Gasvolumen bei einer Temperatur von 20°C und bei einem Druck von 100 kPa.
Für die Testung der katalytischen Aktivität des gemäß Beispiel 1 hergestellten Kupferchromit-Katalysators kam eine rohe Hydroxypivalinaldehydlösung zum Einsatz, die durch Aldolreaktion von iso-Butyraldehyd mit Formaldehyd unter Katalyse von Tri-n-propylamin hergestellt wurde. Das Rohgemisch wurde auf einen bei 150°C und unter Normaldruck betriebenen Verdampfer gegeben. Der flüchtige Verdampferabzug wies folgende Zusammensetzung auf und wurde ohne weitere Reinigung zusammen mit Wasserstoff am Kopf des Rohrreaktors kontinuierlich in die Hydrierstufe gegeben:

Zusammensetzung des Hydriereinsatzes, Angaben in Prozent

| | |
|---|---|
| Formaldehyd | 0,9 |
| Isobutyraldehyd | 1,0 |
| Tri-n-propylamin | 7,0 |
| Isobutanol | 14,5 |
| Hydroxypivalinaldehyd | 48,2 |
| Neopentylglykol | 1,6 |
| Tischtschenko-Ester | 2,7 |

(fortgesetzt)

| Wasser | 24,1 |
|---|---|
| Tischtschenko-Ester: Neopentylglykolmonohydroxypivalinsäureester | |

Über den Sumpf des Rohrreaktors entnahm man das Hydriergut, leitete es in einen Hochdruckabscheider und führte es daraus über eine Standregelung in eine drucklose Vorlage. Die Hydriertemperatur und die Katalysatorbelastung wurden gemäß den Bedingungen der nachfolgenden Tabelle 1 eingestellt. Sämtliche Versuche wurden bei 80 kPa Überdruck durchgeführt.

Umsatz und Selektivität wurde mit Hilfe folgender Formeln bestimmt:

$$\text{Umsatz} = ((\text{Stoffmenge Hydroxypivalinaldehyd im Ausgangsgemisch} - \text{Stoffmenge Hydroxypivalinaldehyd im Hydrieraustrag}) / \text{Stoffmenge Hydroxypivalinaldehyd im Ausgangsgemisch}) * 100$$

$$\text{Selektivität} = (\text{Stoffmenge Neopentylglykol im Hydrieraustrag} / (\text{Stoffmenge Hydroxypivalinaldehyd im Ausgangsgemisch} - \text{Stoffmenge Hydroxypivalinaldehyd im Hydrieraustrag})) * 100$$

Tabelle 1: Kontinuierliche Gasphasenhydrierung von Hydroxypivalinaldehyd am barium- und mangandotierten Kupferchromit-Katalysator gemäß Beispiel 1

| Temperatur/°C | V/Vh/h | Umsatz Hydroxypivalinaldehyd/% | Selektivität zu Neopentylglykol/% |
|---|---|---|---|
| 161 | 0,29 | 99,9 | 97,3 |
| 163 | 0,48 | 99,9 | 98,2 |
| 155 | 0,57 | 99,8 | 98,6 |

Wie die Versuchsergebnisse zeigen, liefert die kontinuierliche Gasphasenhydrierung von Hydroxypivalinaldehyd in Gegenwart eines barium- und mangandotierten Kupferchromit-Katalysators hinsichtlich Umsatz und Selektivität zu Neopentylglykol ausgezeichnete Ergebnisse.

**Beispiel 3: Untersuchung der Formaldehyd-Toleranz des Katalysators aus Beispiel 1**

Es wurde der Versuchsaufbau wie in Beispiel 2 verwendet. Durch gezielte Zugabe einer 40%-igen wässrigen Formaldehydlösung erhielt man Ausgangsgemische mit nachstehender Zusammensetzung , die anschließend bei 155°C, 80 kPa Überdruck und einer V/Vh von 0,3 h$^{-1}$ hydriert wurden (Angaben in Prozent).

| Ausgangsgemisch | 1 | 2 | 3 |
|---|---|---|---|
| Formaldehyd | 1,4 | 0,9 | 1,1 |
| Isobutyraldehyd | 1,0 | 1,0 | 0,7 |
| Tri-n-propylamin | 7,0 | 7,0 | 6,1 |
| Isobutanol | 14,4 | 14,5 | 15,0 |
| Hydroxypivalinaldehyd | 48,1 | 48,2 | 48,2 |
| Neopentylglykol | 1,6 | 1,6 | 1,8 |
| Tischtschenko-Ester | 2,8 | 2,7 | 2,9 |

(fortgesetzt)

| Ausgangsgemisch | 1 | 2 | 3 |
|---|---|---|---|
| Wasser | 23,7 | 24,1 | 24,2 |

Dabei wurden folgende Ergebnisse erzielt:

| Ausgangsgemisch | Umsatz Hydroxypivalinaldehyd/% | Selektivität zu Neopentylglykol/% |
|---|---|---|
| 1 | 99,8 | 98,5 |
| 2 | 99,8 | 98,2 |
| 3 | 99,9 | 97,8 |

## Patentansprüche

1. Kontinuierliches Verfahren zur Herstellung von Neopentylglykol durch Addition von iso-Butyraldehyd und Formaldehyd in Gegenwart eines tertiären Alkylamins als Katalysator zum Hydroxypivalinaldehyd mit nachfolgender Hydrierung in der Gasphase bei einer Temperatur von 125 bis 180°C, **dadurch gekennzeichnet, dass** die Hydrierung in Gegenwart eines Kupferchromit-Katalysators enthaltend die Aktivatoren Barium und Mangan und bei einem Überdruck von 30 bis 120 kPa erfolgt.

2. Verfahren gemäß Anspruch 1, **dadurch gekennzeichnet, dass** die Hydrierung in Gegenwart von Wasser in einer Menge von 15 bis 25 Gew.-%, vorzugsweise von 18 bis 25 Gew.-%, bezogen auf die gesamte Einsatzmenge erfolgt.

3. Verfahren gemäß Anspruch 1 oder 2, **dadurch gekennzeichnet, dass** die Hydrierung bei einer Temperatur von 140 bis 180°C und bei einem Überdruck von 60 bis 100 kPa durchgeführt wird.

4. Verfahren gemäß einem oder mehreren der Ansprüche 1 bis 3, **dadurch gekennzeichnet, dass** man als tertiäres Alkylamin symmetrische tertiäre Alkylamine verwendet.

5. Verfahren gemäß Anspruch 4, **dadurch gekennzeichnet, dass** man als symmetrische tertiäre Alkylamine Trimethylamin, Triethylamin, Tri-n-propylamin oder Tri-n-butylamin verwendet.

6. Verfahren gemäß einem oder mehreren der Ansprüche 1 bis 3, **dadurch gekennzeichnet, dass** man als tertiäres Alkylamin unsymmetrische tertiäre Alkylamine oder Verbindungen mit mehreren Trialkylaminfunktionen verwendet.

7. Verfahren gemäß einem oder mehreren der Ansprüche 1 bis 6, **dadurch gekennzeichnet, dass** der Kupferchromit-Katalysator Barium in einer Menge von 0,5 bis 8 Gew.-% und Mangan in einer Mengen von 0,5 bis 8 Gew.-%, jeweils bezogen auf die Summe des Gehalts an Kupfer, Chrom, Barium und Mangan, enthält.

8. Verfahren gemäß Anspruch 7, **dadurch gekennzeichnet, dass** der Kupferchromit-Katalysator Barium in einer Menge von 1 bis 4 Gew.-% und Mangan in einer Menge von 3 bis 5 Gew.-%, jeweils bezogen auf die Summe des Gehalts an Kupfer, Chrom, Barium und Mangan, enthält.

9. Verfahren gemäß einem oder mehreren der Ansprüche 1 bis 8, **dadurch gekennzeichnet, dass** die Hydrierung bei einem Verhältnis von Kreisgasstrom zu Frischgasstrom von 40 bis 70 durchgeführt wird.

## Claims

1. Continuous method for preparing neopentyl glycol by addition of isobutyraldehyde and formaldehyde in the presence of a tertiary alkylamine as catalyst to give hydroxypivalaldehyde with subsequent hydrogenation in the gas phase at a temperature of 125 to 180°C, **characterised in that** the hydrogenation is carried out in the presence of a copper chromite catalyst comprising the activators barium and manganese and at a superatmospheric pressure of 30 to 120 kPa.

**2.** Method according to Claim 1, **characterised in that** the hydrogenation is carried out in the presence of water in an amount of 15 to 25% by weight, preferably 18 to 25% by weight, based on the total amount used.

**3.** Method according to Claim 1 or 2, **characterised in that** the hydrogenation is carried out at a temperature of 140 to 180°C and a superatmospheric pressure of 60 to 100 kPa.

**4.** Method according to one or more of Claims 1 to 3, **characterised in that** the tertiary alkylamines used are symmetrical tertiary alkylamines.

**5.** Method according to Claim 4, **characterised in that** the symmetrical tertiary alkylamines used are trimethylamine, triethylamine, tri-n-propylamine or tri-n-butylamine.

**6.** Method according to one or more of Claims 1 to 3, **characterised in that** the tertiary alkylamines used are asymmetrical tertiary alkylamines or compounds having a plurality of trialkylamine functions.

**7.** Method according to one or more of Claims 1 to 6, **characterised in that** the copper chromite catalyst comprises barium in an amount of 0.5 to 8% by weight and manganese in an amount of 0.5 to 8% by weight, in each case based on the total content of copper, chromium, barium and manganese.

**8.** Method according to Claim 7, **characterised in that** the copper chromite catalyst comprises barium in an amount of 1 to 4% by weight and manganese in an amount of 3 to 5% by weight, in each case based on the total content of copper, chromium, barium and manganese.

**9.** Method according to one or more of Claims 1 to 8, **characterised in that** the hydrogenation is carried out at a ratio of cycle gas stream to fresh gas stream of 40 to 70.

## Revendications

**1.** Procédé continu de fabrication de néopentylglycol par addition d'iso-butyraldéhyde et de formaldéhyde en présence d'une alkylamine tertiaire en tant que catalyseur à l'hydroxypivalinaldéhyde avec hydrogénation ultérieure dans la phase gazeuse à une température de 125 à 180 °C, **caractérisé en ce que** l'hydrogénation a lieu en présence d'un catalyseur de chromite de cuivre contenant les activateurs baryum et manganèse et à une surpression de 30 à 120 kPa.

**2.** Procédé selon la revendication 1, **caractérisé en ce que** l'hydrogénation a lieu en présence d'eau en une quantité de 15 à 25 % en poids, de préférence de 18 à 25 % en poids, par rapport à la quantité utilisée totale.

**3.** Procédé selon la revendication 1 ou 2, **caractérisé en ce que** l'hydrogénation est réalisée à une température de 140 à 180 °C et à une surpression de 60 à 100 kPa.

**4.** Procédé selon une ou plusieurs des revendications 1 à 3, **caractérisé en ce que** des alkylamines tertiaires symétriques sont utilisées en tant qu'alkylamine tertiaire.

**5.** Procédé selon la revendication 4, **caractérisé en ce que** de la triméthylamine, de la triéthylamine, de la tri-n-propylamine ou de la tri-n-butylamine est utilisée en tant qu'alkylamine tertiaire symétrique.

**6.** Procédé selon une ou plusieurs des revendications 1 à 3, **caractérisé en ce que** des alkylamines tertiaires asymétriques ou des composés contenant plusieurs fonctions trialkylamine sont utilisés en tant qu'alkylamine tertiaire.

**7.** Procédé selon une ou plusieurs des revendications 1 à 6, **caractérisé en ce que** le catalyseur de chromite de cuivre contient du baryum en une quantité de 0,5 à 8 % en poids et du manganèse en une quantité de 0,5 à 8 % en poids, à chaque fois par rapport à la somme de la teneur en cuivre, chrome, baryum et manganèse.

**8.** Procédé selon la revendication 7, **caractérisé en ce que** le catalyseur de chromite de cuivre contient du baryum en une quantité de 1 à 4 % en poids et du manganèse en une quantité de 3 à 5 % en poids, à chaque fois par rapport à la somme de la teneur en cuivre, chrome, baryum et manganèse.

**9.** Procédé selon une ou plusieurs des revendications 1 à 8, **caractérisé en ce que** l'hydrogénation est réalisée à un rapport entre le courant de gaz circulaire et le courant de gaz frais de 40 à 70.

**IN DER BESCHREIBUNG AUFGEFÜHRTE DOKUMENTE**

**In der Beschreibung aufgeführte Patentdokumente**

- EP 0278106 A1
- US 4094014 A
- EP 0484800 A2
- US 4855515 A
- WO 9829374 A1
- DE 1518784 A1
- EP 0006460 A1
- GB 2482887 A
- EP 0522368 A1
- DE 102008033163 A1

**In der Beschreibung aufgeführte Nicht-Patentliteratur**

- Ullmann's Encyclopedia of Industrial Chemistry, VCH Verlagsgesellschaft. 1985, vol. A1, 308
- **JAHRGANG.** *Chemiker-Zeitung,* 1976, vol. 100 (12), 504-514